# EUROPÄISCHE PATENTSCHRIFT

(11) **EP 3 515 529 B1**
(45) Veröffentlichungstag und Bekanntmachung des Hinweises auf die Patenterteilung: **24.07.2024**
(21) Anmeldenummer: 17777230.8
(22) Anmeldetag: 22.09.2017
(51) Int. Cl.: A61M 1/34, A61M 1/36, A61M 5/14, A61M 39/10

(54) **EINSATZSTÜCK FÜR EINEN BLUTSCHLAUCHSATZ ZUM BEGÜNSTIGEN EINER VERMISCHUNG EINER INFUSIONSLÖSUNG MIT EINEM WEITEREN FLUID**
INSERT PIECE FOR A BLOOD LINE SET FOR PROMOTING THE MIXTURE OF AN INFUSION SOLUTION WITH ANOTHER FLUID
INSERT POUR UN ENSEMBLE DE TUBULURES SANGUINES PERMETTANT DE FACILITER UN MÉLANGE D'UNE SOLUTION D'INFUSION AVEC UN AUTRE LIQUIDE

(30) Priorität: 23.09.2016 DE 102016117974
(43) Veröffentlichungstag der Anmeldung: 31.07.2019
(73) Patentinhaber: Fresenius Medical Care Deutschland GmbH, 61346 Bad Homburg (DE)
(72) Erfinder: HÄCKER, Jürgen, 61267 Neu-Anspach (DE); KLEWINGHAUS, Jürgen, 61440 Oberursel (DE)
(74) Vertreter: Bobbert & Partner Patentanwälte PartmbB
(86) Internationale Anmeldenummer: PCT/EP2017/074043
(87) Internationale Veröffentlichungsnummer: WO 2018/055091

(56) Entgegenhaltungen:
- WO-A1-2014/026771
- WO-A1-2016/206804
- US-A- 5 306 265
- US-A1- 2010 168 643

## Beschreibung

Die Erfindung betrifft ein Einsatzstück gemäß dem Oberbegriff des Anspruchs 1, einen extrakorporalen Blutschlauchsatz gemäß dem Oberbegriff des Anspruchs 10 und eine Blutbehandlungsvorrichtung gemäß dem Oberbegriff des Anspruchs 15.

Bei der extrakorporalen Blutbehandlung werden Infusionslösungen oder Medikamente meist über den extrakorporalen Blutkreislauf, d. h. das verwendete Blutschlauchsystem, infundiert. Je nach Art der Infusionslösung kann eine schnelle Mischung der Infusionslösung mit dem Blut wünschenswert sein.

So werden bei der extrakorporalen Blutbehandlung standardmäßig Infusionslösungen zur Hemmung der Blutgerinnung infundiert, um einem möglichen Verschluss des extrakorporalen Blutkreislaufs vorzubeugen.

Dazu werden hauptsächlich zwei Verfahren angewendet, die systemische und die regionale Antikoagulation. Bei der regionalen Antikoagulation wird als Antikoagulanz meist eine Citratlösung verwendet, die Calcium komplexiert und so die Gerinnung des Blutes unterdrückt. Vor Rückgabe des Bluts an den Patienten muss Calcium substituiert werden, da zu niedrige Calciumkonzentrationen Auswirkungen auf Nerven und Muskeln, die Blutgerinnung sowie Funktion von Lunge, Herz und Nieren haben. Deshalb wird bei der regionalen Antikoagulation dem Blut vor Reinfusion in den Patienten eine calciumhaltige Lösung zudosiert, durch die die physiologische Calciumkonzentration im systemischen Blut dauerhaft aufrechterhalten werden kann.

Die Infusion von Infusionslösungen oder Medikamenten in den Schlauchsatz erfolgt üblicherweise über Zugabestellen in T-Form, sogenannten T-Stücken. In diesen T-Stücken herrschen aufgrund des kreisförmigen Querschnitts und der glatten Innenwandung an der Zugabestelle laminare Strömungsbedingungen vor. Zusätzlich sind die Flussraten der infundierten Lösungen im Vergleich zum Blutfluss gering.

Die weitgehend laminaren Strömungsbedingungen und die geringen Flüsse der in den Blutstrom einfließenden Infusionslösungen oder Medikamente können eine zeitlich verzögerte oder nur langsame Durchmischung des Blutes mit der zugegebenen Infusionslösung bedingen. Diese langsame Vermischung der beiden Flüssigkeiten an der Zugabestelle ist speziell bei der Zugabe der Calciumlösung zu Blut unerwünscht und kann aufgrund punktuell dauerhafter hoher Calciumkonzentrationen im Blut vereinzelt zu Gerinnungsproblemen an der Zugabestelle führen. Um dies zu verhindern, ist die rasche und homogene Durchmischung der zugegebenen Calciumlösung mit dem Blut wünschenswert.

Dasselbe Problem kann sich aber auch bei der Zumischung anderer Flüssigkeiten ergeben, z. B. Medikamenten, deren schnelle Durchmischung ebenfalls vorteilhaft sein kann.

Im Stand der Technik ist dieses Problem bekannt. Zu seiner Lösung wurden spezielle Blutschlauchsätze, die Mittel zur Turbulenzerzeugung enthalten, beschrieben.

So ist in der WO 2014/026771 A1 ein Einsatzstück in Gestalt eines T-Stücks für einen Blutschlauchsatz beschrieben, welches eine Spiralstruktur aufweist. Die Spiralstruktur dient dem Erzeugen von Turbulenzen im Bereich der Infusionsstelle. Die Turbulenzen tragen zu einer besseren Vermischung der zusammengeführten Fluide bei.

Ferner sind Lösungen bekannt, die durch intermittierenden Betrieb einer Infusionspumpe einen gepulsten Infusionsstrom der Infusionslösung in das Blut hinein erzeugen.

Eine Aufgabe kann darin bestehen, eine weitere Lösung zum Begünstigen einer Vermischung einer Infusionslösung mit einem weiteren Fluid, etwa Blut, vorzuschlagen.

Die Aufgabe kann gelöst werden durch ein Einsatzstück mit den Merkmalen des Anspruchs 1, einen extrakorporalen Blutschlauchsatz mit den Merkmalen des Anspruchs 10 sowie eine Blutbehandlungsvorrichtung mit den Merkmalen des Anspruchs 15.

Das erfindungsgemäße Einsatzstück dient dazu, in einen Blutschlauchsatz (oder in ein Blutschlauchsystem) eingesetzt zu werden oder Teil hiervon zu sein. Das Einsatzstück umfasst wenigstens eine erste Anschlussstelle zum Anschließen eines ersten Schlauchabschnitts des Blutschlauchsatzes an das Einsatzstück. Es umfasst eine zweite Anschlussstelle zum Anschließen eines zweiten Schlauchabschnitts des Blutschlauchsatzes an das Einsatzstück. Es umfasst eine dritte Anschlussstelle zum Anschließen eines dritten Schlauchabschnitts des Blutschlauchsatzes an das Einsatzstück. Es umfasst eine Hauptleitung zum Leiten einer ersten Flüssigkeit, vorzugsweise Blut, durch das Einsatzstück hindurch. Die Hauptleitung steht in Fluidverbindung mit zumindest der ersten Anschlussstelle und mit der zweiten Anschlussstelle oder jeweils einem von der ersten Anschlussstelle und/oder mit der zweiten Anschlussstelle umgebenen oder von dieser gebildeten Lumen.

Das Einsatzstück umfasst wenigstens eine Nebenleitung zum Leiten einer zweiten Flüssigkeit, vorzugsweise einer Infusionslösung, direkt oder indirekt, in die Hauptleitung hinein. Die Nebenleitung steht in Fluidverbindung mit der dritten Anschlussstelle oder einem von dieser umgebenen Lumen. Die Nebenleitung steht in einem Zwischenabschnitt des Einsatzstücks, welcher zwischen der ersten Anschlussstelle und mit der zweiten Anschlussstelle angeordnet, in Fluidverbindung mit der Hauptleitung.

Die erste Anschlussstelle weist ein durchströmbares Lumen mit einer ersten Querschnittsfläche auf. Die zweite Anschlussstelle weist ein durchströmbares Lumen mit einer zweiten Querschnittsfläche auf. Der Zwischenabschnitt weist ein durchströmbares Lumen mit einer dritten Querschnittsfläche auf. Die Nebenleitung weist einen Lumenabschnitt auf. Der Lumenabschnitt weist wenigstens eine Mündung oder Austrittsöffnung für das zweite Fluid auf. Der Lumenabschnitt ragt in das Innere des Zwischenabschnitts hinein.

Der erfindungsgemäße Blutschlauchsatz, extrakorporale Blutkreislauf oder Blutschlauchsystem weist wenigstens ein erfindungsgemäßes Einsatzstück auf.

In einigen beispielhaften, erfindungsgemäßen Ausführungsformen ist das erfindungsgemäße Einsatzstück zwischen Schlauchabschnitte des Blutschlauchsatzes eingeschoben oder integral mit diesen gefertigt.

In einigen beispielhaften, erfindungsgemäßen Ausführungsformen ist das erfindungsgemäße Einsatzstück unlösbar mit Schlauchabschnitten des Blutschlauchsatzes verbunden, in anderen lösbar.

Die erfindungsgemäße Blutbehandlungsvorrichtung ist mit wenigstens einem erfindungsgemäßen Blutschlauchsatz verbunden.

Erfindungsgemäße Ausführungsformen können eines oder mehrere der vorstehend oder im Folgenden genannten Merkmale in beliebiger Kombination aufweisen, jeweils basierend auf einem der unabhänigen Ansprüche. Erfindungsgemäße Ausführungsformen sind ferner Gegenstand der Unteransprüche.

Bei allen hierin gemachten Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Hierin gemachte räumliche Angaben wie "oben", "unten", usw. beziehen sich im Zweifel auf die Darstellung, wie sie den hier beigefügten Figuren zu entnehmen ist.

In bestimmten, beispielhaften erfindungsgemäßen Ausführungsformen mündet die Austrittsöffnung in einem (in radialer Richtung) zentralen Bereich des durchströmbaren Lumens oder in einem zentralen Bereich des Zwischenabschnitts.

In bestimmten, beispielhaften erfindungsgemäßen Ausführungsformen weist die erste Anschlussstelle, die zweite Anschlussstelle und/oder der Zwischenabschnitt jeweils eine weitgehend zylinderförmige Innenwand oder einen Abschnitt mit einer zylinderförmigen Innenwand auf.

In bestimmten, beispielhaften erfindungsgemäßen Ausführungsformen ist das Einsatzstück integral mit dem Blutschlauchsatz gefertigt. Alle oder manche der hierin als Anschlussstellen bezeichneten Abschnitte des Einsatzstücks sind in solchen Ausführungsformen Übergangsabschnitte zwischen Einsatzstück und angrenzenden oder weiterführenden Schlauchabschnitten.

In bestimmten, beispielhaften erfindungsgemäßen Ausführungsformen ist die Nebenleitung in Fluidverbindung mit einer Quelle für die Infusionslösung verbunden oder weist diese auf.

In bestimmten, beispielhaften erfindungsgemäßen Ausführungsformen ist die Infusionslösung eine Calciumlösung oder weist eine Calciumlösung auf. Die Erfindung ist jedoch nicht auf die Verwendung einer calciumhaltigen Lösung beschränkt. Andere Medikamentenlösungen sind ebenfalls von der vorliegenden Erfindung umfasst. Zu ihnen zählt insbesondere eine Citratlösung.

In bestimmten, beispielhaften erfindungsgemäßen Ausführungsformen ist die dritte Querschnittsfläche so groß oder im Wesentlichen so groß wie die erste Querschnittsfläche und/oder die zweite Querschnittsfläche. Als "im Wesentlichen so groß" kann ein Wert von 80 %, 90 % oder mehr gelten. Alle Zwischenwerte, und insbesondere jeder ganzzahlige Prozentwert (81 %, 82 %, 83 %, usw.), sind ebenfalls umfasst.

In bestimmten, beispielhaften erfindungsgemäßen Ausführungsformen ist der Lumenabschnitt derart ausgestaltet, dass die zweite Flüssigkeit aus der Austrittsöffnung in oder im Wesentlichen in einer axialen Richtung der Hauptleitung oder parallel zu einer Hauptströmungsrichtung der Hauptleitung in letztere abgegeben wird oder abgegeben werden kann. Diese Ausgestaltung kann vor allem die im Zwischenabschnitt vorliegende Hauptströmungsrichtung betreffen. Dies kann beispielsweise durch einen Knick der Nebenleitung im Zwischenabschnitt erzielt werden.

In bestimmten, beispielhaften erfindungsgemäßen Ausführungsformen ist die Austrittsöffnung angeordnet, um die zweite Flüssigkeit in oder im Wesentlichen in einer axialen Richtung der Hauptleitung oder parallel zu einer Hauptströmungsrichtung der Hauptleitung in letztere abzugeben. Hierbei kann vorzugsweise die im bestimmungsgemäßen Gebrauch des Einsatzstücks im Zwischenabschnitt vorliegende Hauptströmungsrichtung gemeint sein.

In bestimmten, beispielhaften erfindungsgemäßen Ausführungsformen ist ein sich an die Austrittsöffnung für die zweite Flüssigkeit stromaufwärts - bezogen auf die zweite Flüssigkeit, wenn diese Richtung Hauptleitung strömt - anschließender Abschnitt des Lumenabschnitts vorgesehen. Er ist derart angeordnet, dass sich seine Längserstreckung oder seine Längsachse parallel, oder im Wesentlichen parallel, zur Längsachse des Zwischenabschnitts oder parallel zu einer Mittellinie der Hauptleitung erstreckt.

In bestimmten, beispielhaften erfindungsgemäßen Ausführungsformen ist im Bereich des Zwischenabschnitts ein Prall- oder Umlenkelement im Inneren des Zwischenabschnitts vorgesehen. Dieses Element ist derart angeordnet ist, dass eine aus der Austrittsöffnung oder Mündung der Nebenleitung austretende zweite Flüssigkeit in ihrer radialen Bewegung oder in ihrer Bewegung in Austrittsrichtung begrenzt ist.

In bestimmten, beispielhaften erfindungsgemäßen Ausführungsformen ist das Prall- oder Umlenkelement nicht die Innenwandung der Hauptleitung, etwa im Bereich der ersten Anschlussstelle, der zweiten Anschlussstelle oder des Zwischenabschnitts.

In bestimmten, beispielhaften erfindungsgemäßen Ausführungsformen liegt das radial innere Ende des Prall- oder Umlenkelements weiter radial innen als die Innenwandung der Hauptleitung, etwa im Bereich der ersten Anschlussstelle, der zweiten Anschlussstelle oder des Zwischenabschnitts.

In bestimmten, beispielhaften erfindungsgemäßen Ausführungsformen ist das Prall- oder Umlenkelement Teil des Lumenabschnitts.

In bestimmten, beispielhaften erfindungsgemäßen Ausführungsformen erstreckt sich der Lumenabschnitt einstückig oder mehrstückig durch das gesamte Innere der Hauptleitung hindurch, und zwar in Querrichtung oder in radialer Richtung.

In bestimmten, beispielhaften erfindungsgemäßen Ausführungsformen erstreckt sich im Bereich des Zwischenabschnitts ein Vorsprung in das Innere des Zwischenabschnitts hinein. Der Vorsprung liegt in einer Verlängerung oder in einer gedachten Fortführung der Nebenleitung im Inneren des Zwischenabschnitts.

In bestimmten, beispielhaften erfindungsgemäßen Ausführungsformen ist eine Zugabeleitung mittels der Nebenleitung des Einsatzstücks in Fluidkommunikation mit einer Blutrückgabeleitung und/oder mit einer Blutentnahmeleitung des Blutschlauchsatzes verbunden.

In bestimmten, beispielhaften erfindungsgemäßen Ausführungsformen ist der Blutschlauchsatz für die Durchführung einer regionalen Antikoagulation geeignet und/oder vorbereitet.

In bestimmten, beispielhaften erfindungsgemäßen Ausführungsformen ist der Blutschlauchsatz zur Durchführung einer Hämodialyse, einer Hämofiltration oder einer Hämodiafiltration oder einer Plasmapheresebehandlung oder Vollblutadsorptionsbehandlung geeignet und/oder vorbereitet.

In bestimmten, beispielhaften erfindungsgemäßen Ausführungsformen ist der Lumenabschnitt kein Abschnitt, der an der Innenwandung des Zwischenabschnitts anliegt oder der die Innenwandung fortsetzten würde, insbesondere anliegen oder festsetzen würde, ohne dabei einen radialen Absatz oder eine radiale Stufe zu bilden.

Erfindungsgemäß ragt der Lumenabschnitt über die Innenwandung hinaus und in das Innere des Zwischenabschnitts hinein.

In bestimmten, beispielhaften erfindungsgemäßen Ausführungsformen weist der Lumenabschnitt - in einer Ansicht eines Querschnitts des Zwischenabschnitts - keine Ringform auf.

In bestimmten, beispielhaften erfindungsgemäßen Ausführungsformen ist der Lumenabschnitt keine blendenförmige Struktur.

In bestimmten, beispielhaften erfindungsgemäßen Ausführungsformen liegt der Lumenabschnitt nicht - z. B. in einem Querschnitt des Zwischenabschnitts - der Innenwandung des Zwischenabschnitts kreisförmig, also über dem gesamten Innenumfang des Zwischenabschnitts, an.

In bestimmten, beispielhaften erfindungsgemäßen Ausführungsformen verengt der Lumenabschnitt das durchströmbare Lumen des Zwischenabschnitts nicht kreisförmig, also über dem gesamten Innenumfang des Zwischenabschnitts.

In bestimmten, beispielhaften erfindungsgemäßen Ausführungsformen ist der Lumenabschnitt im Inneren der Hauptleitung derart angeordnet, dass Flüssigkeit, welche das Lumen der Hauptleitung durchströmt, im Bereich des Lumenabschnitts vor diesem (also vor der Zeichenebene der hier angehängten Zeichnung) als auch hinter diesem (also hinter der Zeichenebene der hier angehängten Zeichnung) strömen kann.

In bestimmten, beispielhaften erfindungsgemäßen Ausführungsformen ragt der Lumenabschnitt stiftförmig oder zylinderförmig in das Lumen der Hauptleitung hinein.

In bestimmten, beispielhaften erfindungsgemäßen Ausführungsformen weist der Lumenabschnitt wenigstens eine Rille auf, welche sich zumindest abschnittsweise in einer Richtung parallel zur Hauptleitung erstreckt.

Die Rille kann aus Fertigungsgründen vorteilhaft ausschließlich gerade verlaufen. Sie kann zur besseren Verteilung des durch die Nebenleitung zugeführten Fluids einen gekrümmten Abschnitt in Längsrichtung haben.

Die Rille kann in einem Querschnitt eine gekrümmte, eine dreieckige oder jede andere Form haben.

In bestimmten, beispielhaften erfindungsgemäßen Ausführungsformen ist das Einsatzstück aus Kunststoff gefertigt, vorzugweise spritzgegossen.

In bestimmten, beispielhaften erfindungsgemäßen Ausführungsformen weist das Einsatzstück keine dreidimensionale Spiralstuktur zur Erzeugung einer Turbulenz auf, insbesondere keinen vertieften Ausschnitt in der Innenwandung des Zwischenabschnitts.

In bestimmten, beispielhaften erfindungsgemäßen Ausführungsformen ist der Lumenabschnitt nicht als erhöhter Wulst auf der Innenwandung des Zwischenabschnitts ausgestaltet, oder er ist keine Verdickung der Innenwandung.

In einigen, beispielhaften erfindungsgemäßen Ausführungsformen ist die erste, die zweite und/oder die dritte Anschlussstelle mit jeweils einem Schlauchabschnitt verklebt. Alternativ kann die Verbindung eine Steck- oder Klemmverbindung oder eine andere Verbindung sein.

In einigen, beispielhaften, erfindungsgemäßen Ausführungsformen weist der Blutschlauchsatz eine Tropfkammer, weitere Einsatzstücke in Form von T-Stücken oder Zuspritzstellen usw. auf.

In einigen, beispielhaften, erfindungsgemäßen Ausführungsformen ist der Blutschlauchsatz zur Durchführung einer extrakorporalen Blutbehandlung mit einer regionalen Antikoagulation vorgesehen und/oder geeignet.

Wenn hierin von Flüssigkeiten die Rede ist, ist dies nicht beschränkend zu verstehen. Die vorliegende Erfindung umfasst auch das Zusammenführen von Fluiden im weiteren Sinne.

Einige oder alle erfindungsgemäßen Ausführungsformen können einen oder mehrere der oben oder im Folgenden genannten Vorteile aufweisen.

Ein Vorteil besteht darin, dass es aufgrund des erfindungsgemäßen Einspritzens oder Einbringens der Infusionslösung in das Blut keiner weiteren Vorrichtungen oder Verfahrensschritte zum Sicherstellen einer Verwirbelung der Infusionslösung innerhalb des Bluts bedarf. Dennoch kann eine beschleunigte und zuverlässige Durchmischung der Infusionslösung mit dem Blut erzielt werden.

Ein Vorteil der Verwendung des erfindungsgemäßen Einsatzstücks oder des erfindungsgemäßen Blutschlauchsatzes kann ferner darin bestehen, dass die hierin beschriebenen Vorteile erzielbar sein können, ohne dass hierzu eine Veränderung an der Steuerung der Blutbehandlungsvorrichtung oder der Pumpe für die Infusionslösung vorgenommen werden müsste. Denn die Struktur des erfindungsgemäßen Einsatzstücks bewirkt eine vorteilhafte Verteilung des zweiten Fluids im ersten Fluid, ohne dass dies z. B. einer pulsierenden Zugabe bedürfte wie z. B. in der DE 10 2013 011 010 A1 beschrieben. Der erfindungsgemäße Effekt kann allein mittels der hierin beschriebenen Geometrie des Einsatzstücks erzielt werden. Insbesondere bedarf es keiner geänderten Ansteuerung der Infusionspumpe. Ihr Förderverhalten kann beibehalten bleiben.

Damit einher geht vorteilhaft, dass es keiner baulichen oder andersartigen Anpassung bereits ausgelieferter Blutbehandlungsvorrichtungen, etwa einer Softwareanpassung, bedarf. Die Umsetzung der vorliegenden Erfindung kann aus dem Verwenden von erfindungsgemäßen Einsatzstücken oder Blutschlauchsätzen bestehen. Ein Umstellen auf Letztere ist auch deshalb einfach zu bewerkstelligen, da es sich hierbei ohnehin um Einwegartikel oder Disposables handeln wird.

Die Wirkung des erfindungsgemäßen Einsatzstücks ist unabhängig von Parametern der Blutbehandlung. Werden beispielsweise die Förderrate der Blutpumpe und im Einklang hiermit die Förderrate der Pumpe für die Infusionslösung verändert, so ändert dies nichts am Wirkprinzip oder an der Wirkung des erfindungsgemäßen Einsatzstücks.

Schließlich besticht das erfindungsgemäße Einsatzstück durch seine äußerst einfache und bei Wunsch sogar symmetrische Gestaltung. Es lässt sich mittels einfachster Spritzwerkzeuge herstellen, was den Gesamtaufwand seiner Fertigung erheblich senken kann. Spiralige Verläufe und Konturen sind ebenso verzichtbar wie Hinterschneidungen.

Einfache, gerade Verläufe von Wandungen können erfindungsgemäß am Einsatzstück vorherrschen.

In einigen Ausführungsformen weist der Zwischenabschnitt eine Querschnittsfläche auf, die im Verlauf des Zwischenabschnitts von einem Ende zum anderen in einem ersten Bereich monoton ansteigt und in einem zweiten Bereich monoton abnimmt, vorzugsweise steigt im Verlauf die Querschnittsfläche zunächst an und nimmt dann wieder ab.

In einigen Ausführungsformen gehen der erste und der zweite Bereich unmittelbar ineinander über, so dass vorzugsweise die Querschnittsfläche im Verlauf des Zwischenabschnitts zunächst monoton ansteigt und unmittelbar danach monoton abnimmt. In anderen Ausführungsformen sind der erste Bereich und der zweite Bereich des Zwischenabschnitts voneinander getrennt, beispielsweise durch die Nebenleitung und/oder die Prall- oder Umlenkfläche, vorzugsweise ausschließlich durch die Nebenleitung und/oder die Prall- oder Umlenkfläche. Dabei sinkt die Querschnittsfläche im Verlauf des Zwischenabschnitts vorzugsweise nicht unter 80%, besonders vorzugsweise nicht unter 90% der Querschnittsfläche der ersten und/oder zweiten Anschlussstelle ab und/oder steigt vorzugsweise nicht über 120%, besonders vorzugsweise nicht über 110% derselben an.

In einigen Ausführungsformen nimmt der Querschnitt der ersten und/oder zweiten Anschlussstelle in deren jeweiligen Verlauf nicht ab, insbesondere nicht kontinuierlich ab.

In einigen Ausführungsformen sind die erste Anschlussstelle und die zweite Anschlussstelle derart angeordnet, dass sie auf einer Achse liegen. Vorzugsweise ist die Nebenleitung in einem rechten Winkel zu dieser Achse angeordnet.

Erfindungsgemäß sind im Inneren des Einsatzstücks keine Strömungshindernisse vorhanden, mit Ausnahme der Nebenleitung und/oder der optionalen Prall- oder Umlenkfläche.

Die vorliegende Erfindung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- Fig. 1: zeigt ein erfindungsgemäßes Einsatzstück für einen in Fig. 1 nicht gezeigten Blutschlauchsatz in einer ersten exemplarischen Ausführungsform in einem Längsschnitt;
- Fig. 1a: zeigt das erfindungsgemäße Einsatzstück der Fig. 1 in einer geringfügigen Modifikation;
- Fig. 2: zeigt das erfindungsgemäße Einsatzstück der Fig. 1;
- Fig. 3: zeigt das erfindungsgemäße Einsatzstück in einer zweiten exemplarischen Ausführungsform in einem Längsschnitt;
- Fig. 3a: zeigt das erfindungsgemäße Einsatzstück der Fig. 3 in einem Querschnitt;
- Fig. 4: zeigt das erfindungsgemäße Einsatzstück in einer dritten exemplarischen Ausführungsform in einem Längsschnitt;
- Fig. 5: zeigt das erfindungsgemäße Einsatzstück in einer vierten exemplarischen Ausführungsform in einem Längsschnitt; und
- Fig. 6: zeigt einen erfindungsgemäßen Blutschlauchsatz mit einem erfindungsgemäßen Einsatzstück.

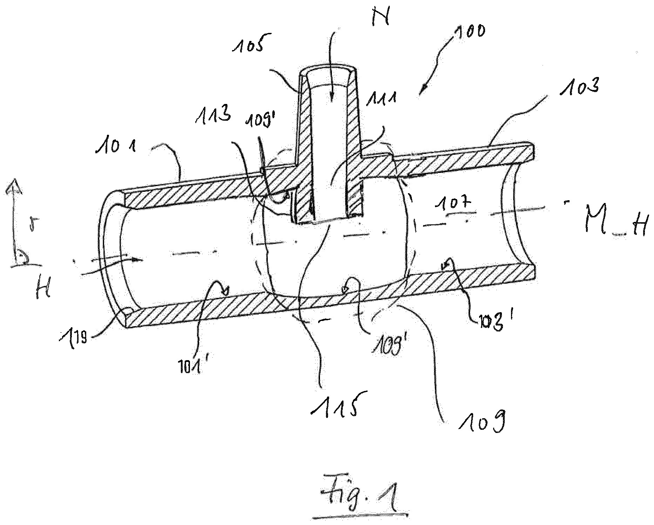

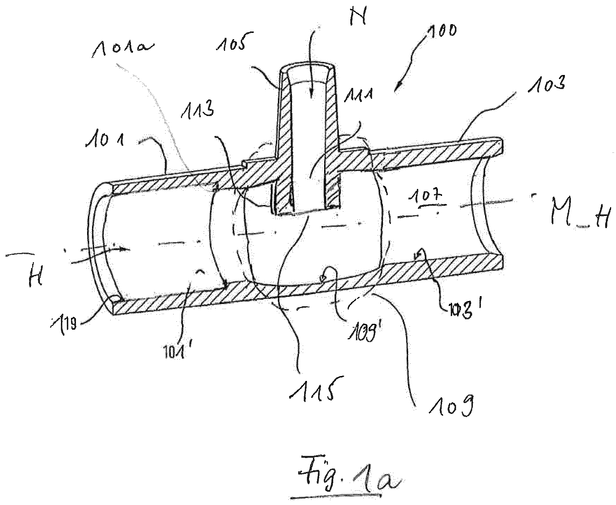

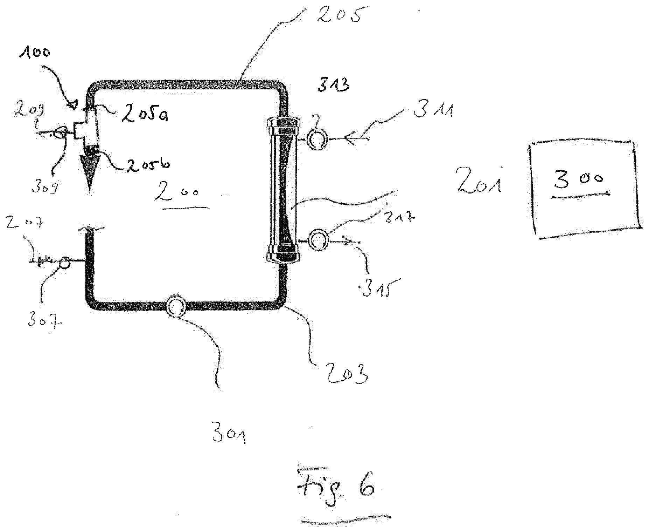

**Fig.** 1 zeigt das erfindungsgemäße Einsatzstück 100 für einen in Fig. 1 nicht gezeigten Blutschlauchsatz 200 (siehe jedoch Fig. 6) in einer ersten exemplarischen Ausführungsform in einem Längsschnitt.

Das Einsatzstück 100 weist eine erste Anschlussstelle 101 auf, mittels welcher ein erster Schlauchabschnitt 205a (siehe Fig. 6) des Blutschlauchsatzes 200 an das Einsatzstück 100 angeschlossen werden kann.

Das Einsatzstück 100 weist eine zweite Anschlussstelle 103 auf, mittels welcher ein zweiter Schlauchabschnitt 205b des Blutschlauchsatzes 200 an das Einsatzstück 100 angeschlossen werden kann.

Das Einsatzstück 100 weist eine dritte Anschlussstelle 105 auf, mittels welcher ein dritter Schlauchabschnitt, hier eine Leitung für Calciumlösung 209 des Blutschlauchsatzes 200, an das Einsatzstück 100 angeschlossen werden kann.

Das Einsatzstück 100 weist eine Hauptleitung 107 zum Leiten einer ersten Flüssigkeit, vorzugsweise Blut, durch das Einsatzstück 100 hindurch auf. Die Hauptleitung 107 steht in Fluidverbindung mit der ersten Anschlussstelle 101 und mit der zweiten Anschlussstelle 103. Die erste Flüssigkeit kann in Richtung des Pfeils H in die Hauptleitung 107 einströmen.

Das Einsatzstück 100 weist eine Längsrichtung auf, welche parallel zur Mittellinie der Hauptleitung 107, welche durch das Bezugszeichen M_H bezeichnet ist, verläuft. Das Einsatzstück 100 weist eine Querrichtung oder radiale Richtung auf, welche mit r bezeichnet ist

Das Einsatzstück 100 weist einen Zwischenabschnitt 109 auf. Die Hauptleitung 107 führt durch ihn hindurch. Der Zwischenabschnitt 109 ist im Fig. 1 mittels Strichlinie umfahren.

Das Einsatzstück 100 weist eine Nebenleitung 111 auf. Sie dient dem Leiten einer zweiten Flüssigkeit, vorzugsweise einer Infusionslösung, in die Hauptleitung 107 hinein. Die zweite Flüssigkeit kann in Richtung des Pfeils N in die Nebenleitung 111 einströmen.

Die Nebenleitung 111 steht in Fluidverbindung mit der dritten Anschlussstelle 105. Sie steht ferner im Zwischenabschnitt 109 des Einsatzstücks 100 in Fluidverbindung mit der Hauptleitung 107.

Die erste Anschlussstelle 101 weist ein durchströmbares Lumen mit einer ersten Querschnittsfläche und einer Innenwandung 101' auf. Die Innenwandung 101' umschließt ein Inneres der ersten Anschlussstelle 101. Das Innere entspricht beim Beispiel der ersten Anschlussstelle 101 deren durchströmbaren Lumen.

Die zweite Anschlussstelle 103 weist ein durchströmbares Lumen mit einer zweiten Querschnittsfläche und einer Innenwandung 103` auf. Die Innenwandung 103' umschließt ein Inneres der zweiten Anschlussstelle 103. Das Innere entspricht beim Beispiel der zweiten Anschlussstelle 103 deren durchströmbaren Lumen.

Der Zwischenabschnitt 109 weist ein durchströmbares Lumen mit einer dritten Querschnittsfläche und einer Innenwandung 109` auf. Die Innenwandung 109' umschließt ein Inneres des Zwischenabschnitts 109. Das Innere entspricht beim Beispiel des Zwischenabschnitts 109 nicht dessen durchströmbaren Lumen. Die Querschnittsfläche des Inneren ist vielmehr um die Querschnittsfläche eines Lumenabschnitts 113, soweit dieser in das Innere hineinreicht, größer als die durchströmbare Querschnittsfläche des Lumens. Denkt man sich in Fig. 1 den Lumenabschnitt 113 weg, so würde in Fig. 1 das Innere dem durchströmbaren Lumen entsprechen.

Die Mittellinie der Hauptleitung 107 (etwa festgelegt durch die Mittelpunkte der ersten und der zweiten Querschnittsflächen, siehe oben) ist mit M_H bezeichnet. Die Mittellinie M_H entspricht der Längsachse der Hauptleitung 107 und deren axialer Richtung.

Die Nebenleitung 111 weist den Lumenabschnitt 113 auf, welcher in das Innere des Zwischenabschnitts 109 ragt und welcher wenigstens eine Mündung oder Austrittsöffnung 115 aufweist. Durch die Austrittsöffnung 115 kann die zweite Flüssigkeit in das Lumen der Hauptleitung 107 eingebracht werden.

Wie Fig. 1 zu entnehmen ist, umschließt die Innenwandung 101' ein durchströmbares Lumen der Hauptleitung 107 im Bereich der ersten Anschlussstelle 101, während die Innenwandung 103' ein durchströmbares Lumen der Hauptleitung 107 im Bereich der zweiten Anschlussstelle 103 umschließt. Die Innenwandung 109` umschließt ein durchströmbares Lumen der Hauptleitung 107 im Bereich des Zwischenabschnitts 109.

Wie Fig. 1 ferner zeigt, ragt der Lumenabschnitt 113 in das Innere des Zwischenabschnitts 109 hinein. Dabei ist der Lumenabschnitt 113 nicht als Verdickung oder Verengung der Wandung des Einsatzstückes zu verstehen, sondern als Durchbrechung oder Fortsetzung der Wandung mit der Absicht, die Austrittsöffnung 115 möglichst weit radial in das durchströmte Innere der Hauptleitung 107 - und damit in den Strom der ersten Flüssigkeit durch die Hauptleitung 107 - vorzuverlagern.

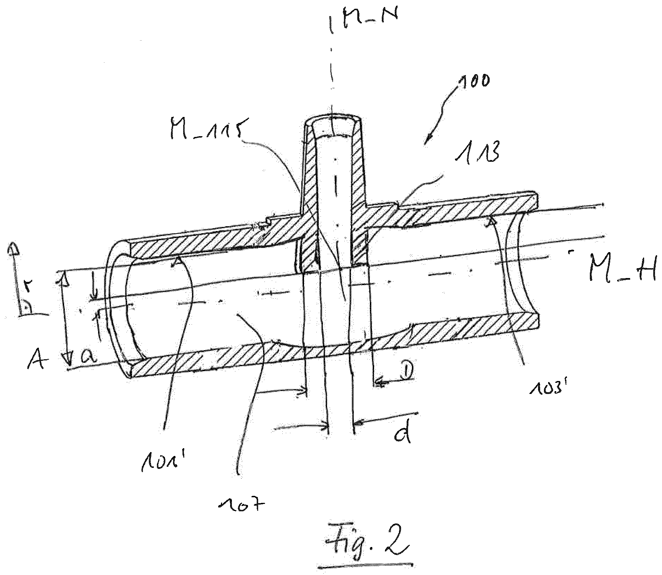

Dies kann dadurch erreicht werden, dass, wie beispielhaft in Fig. 1 gezeigt, der Durchmesser der Austrittsöffnung 115 einen großen Teil des Durchmessers oder der Querschnittsfläche des Lumenabschnitts 113 ausmacht, wie dies mit Bezug auf Fig. 2 weiter erläutert ist.

Die erste Anschlussstelle 101, die zweite Anschlussstelle 103 und/oder die dritte Anschlussstelle 105 können optional jeweils eine Abschrägung oder Fase 119 der Innenwandung oder der Außenwandung aufweisen.

Wie Fig. 1 zu entnehmen ist, handelt es sich beim Lumenabschnitt 113 vorzugsweise nicht um einen Abschnitt, der der Innenwandung 109' des Zwischenabschnitts 109 stufenlos anliegt. Während die Innenwandung 109' das Innere des Zwischenabschnitts 109 begrenzt, ragt der Lumenabschnitt 113 über die Innenwandung 109' hinaus in das Innere des Zwischenabschnitts 109 hinein.

Optional ist der Lumenabschnitt 113 auch keine Struktur, die in einem Querschnitt des Zwischenabschnitts109 ringförmig ist.

Optional ist der Lumenabschnitt 113 keine blendenförmige Struktur, die in einem Querschnitt des Zwischenabschnitts 109 einen Durchlass für das Fluid bildet, an ihrem Außenumfang jedoch der Innenwandung 109` kreisförmig, also beispielsweise über dem gesamten Innenumfang des Zwischenabschnitts 109 anliegen würde.

Optional ist der Lumenabschnitt 113 im Inneren der Hauptleitung 107 derart angeordnet, dass Flüssigkeit, welche das Lumen der Hauptleitung 107 durchströmt, im Bereich des Lumenabschnitts 113 vor diesem (also vor der Zeichenebene) als auch hinter diesem (also hinter der Zeichenebene) strömen kann.

Optional ragt der Lumenabschnitt 113 stiftförmig oder zylinderförmig (z. B. mit der Mittellinie M_N als Drehachse) in das Innere der Hauptleitung 107 hinein.

Ein optionales Merkmal des erfindungsgemäßen Einsatzstücks 100 ist es, dass die durchströmbare Fläche der Hauptleitung 107 im Bereich des Zwischenabschnitts 109 nicht kleiner als die durchströmbare Fläche der Hauptleitung im Bereich der ersten Anschlussstelle 101 und/oder im Bereich der zweiten Anschlussstelle 103 ist. Hier sind erfindungsgemäß verschiedene Ausgestaltungen angedacht. Eine Option ist in Fig. 1 gezeigt; die Innenwandung des Zwischenabschnitts 109 ist verglichen mit benachbarten Abschnitten dünner ausgestaltet, was bei stufenloser Außenwandung eine Vergrößerung des durchströmbaren Lumens zur Folge hat. Alternativ oder ergänzend hierzu kann ein Versatz der Wandung der Hauptleitung 107 im Bereich des Zwischenabschnitts 109 vorgesehen sein, derart, dass die Innenwandung gegenüber benachbarten Bereichen radial nach außen versetzt ist, und dass zudem auch die Außenwandung gegenüber benachbarten Bereichen radial nach außen versetzt ist. Letzteres ist in Fig. 1 nicht gezeigt, ersteres hingegen schon.

**Fig. 1a** zeigt das erfindungsgemäße Einsatzstück 100 der Fig. 1 mit einer Modifikation im linken Schenkel der Hauptleitung 107.

Wie durch das Bezugszeichen 101a gezeigt, weist die Innenwandung 101` der Hauptleitung 107 einen Anschlag auf, gegen welchen ein in Fig. 1a nicht gezeigter Schlauch, welcher in die erste Anschlussstelle 101 eingesteckt ist, geschoben werden kann.

Der hier exemplarisch ringförmige Anschlag 101a kann mit der Stirnfläche des ebenfalls ringförmigen, nicht gezeigten Schlauchs im Gebrauch in Kontakt stehen. Die Höhe des Anschlags 101a kann dabei vorteilhaft so gewählt sein, dass die Innenwandung des Schlauchs mit der Innenwandung 101' des sich an den Anschlag 101a nach rechts anschließenden Abschnitts der Innenwandung 109` stufenlos abschließt. Auf diese Weise wird die Laminarität des Fluids im Stagnationsbereich (nahe der Wandung) nicht ungewünscht beeinträchtigt.

In Fig. 1a ist ein solcher Anschlag 101a, welcher die Form einer Stufe in der Innenwandung 101' haben kann, nur auf der linken Seite des Einsatzstücks 100 gezeigt. Ein solcher Anschlag kann allerdings auch im Bereich der zweiten Anschlussstelle 103 vorgesehen sein.

Ein solcher Anschlag kann ferner in jeder anderen erfindungsgemäßen Ausführungsform vorgesehen sein.

Ein solcher Anschlag kann zudem in jeder erfindungsgemäßen Ausführungsform gemeinsam mit einer Abschrägung oder Fase 119 vorgesehen sein.

**Fig. 2** zeigt das erfindungsgemäße Einsatzstück 100 der Fig. 1. Für eine bessere Übersichtlichkeit wurde in Fig. 2 auf die Wiedergabe einer Vielzahl der aus Fig. 1 bereits bekannten Bezugszeichen verzichtet.

Wie zu erkennen ist ragt der Lumenabschnitt 113 in das Innere des Zwischenabschnitts 109 hinein. Dabei steht die Mündung oder Austrittsöffnung 115 erkennbar über das Niveau der Innenwandung 101' oder 103' in das Innere hinein.

Der Innendurchmesser der Hauptleitung 107 beträgt im Bereich der ersten Anschlussstelle 101 oder im Bereich der zweiten Anschlussstelle 103 den Wert A. Der Mittelpunkt M_115 (oder der geometrische Schwerpunkt) einer Öffnungsebene der Austrittsöffnung 115 liegt um den Absatz a von der Mittellinie M_H entfernt. Dabei beträgt das Verhältnis a/A vorzugweise höchstens 0,33.

Der Außendurchmesser der Nebenleitung 111 beträgt im Bereich der Austrittsöffnung 115 den Wert D. Der Innendurchmesser der Nebenleitung 111 beträgt im Bereich der Austrittsöffnung 115 den Wert d. Dabei beträgt das Verhältnis d/D vorzugweise mindestens 0,125.

Ganz bevorzugt beträgt das Verhältnis a/A vorzugweise höchstens 0,17, gemessen innerhalb eines Bereichs, dessen Punkte höchstens um den Wert 2*d von einer Mittellinie M_N der Nebenleitung 111 entfernt liegen.

Durch die Austrittsöffnung 115 kann die zweite Flüssigkeit in das Lumen der Hauptleitung 107 eingebracht werden.

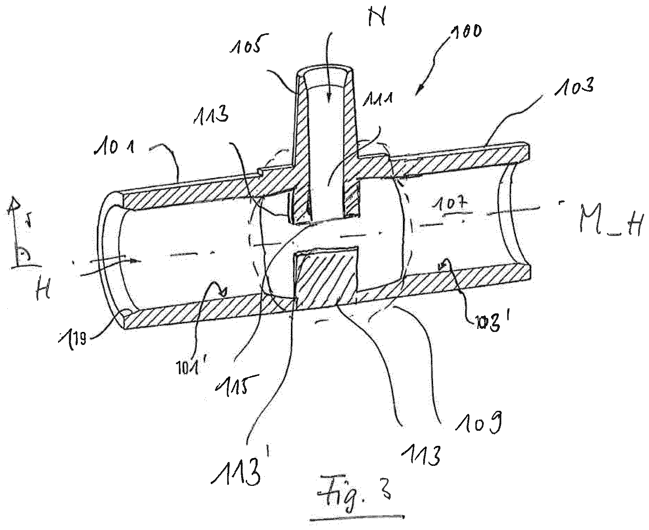

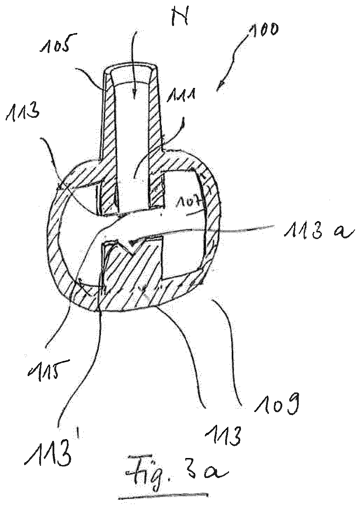

**Fig. 3** zeigt das erfindungsgemäße Einsatzstück 100 für einen in Fig. 3 nicht gezeigten Blutschlauchsatz 200 in einer zweiten exemplarischen Ausführungsform in einem Längsschnitt.

Wie Fig. 3 zu entnehmen ist umfasst der Lumenabschnitt 113 Anteile, die bis an beide gegenüberliegende Innenwandabschnitte der Hauptleitung 107 reichen oder sich bis zu diesen jeweils erstrecken.

Im Beispiel der Fig. 3 liegt der Austrittsöffnung 115 eine Prallfläche oder Umlenkfläche 113' gegenüber. Die Umlenkfläche 113' ist Teil des Lumenabschnitts 113 oder kann Teil hiervon sein. Die Umlenkfläche 113' kann dazu dienen, dass aus der Austrittsfläche 115 austretende zweite Flüssigkeit bei ihrem Austritt aus der Austrittsfläche 115 nicht bis in radial außen gelegene Strömungsbereiche ("Stagnationsbereiche") der Hauptleitung 107 ausgebracht wird, sondern sich näher an der Mittellinie M_H, also in einem Bereich besonders hoher Strömungsgeschwindigkeit, in den Strom der ersten Flüssigkeit hinein begibt.

Die Ausgestaltung der Fig. 3 stellt eine symmetrischere Ausgestaltung des Einsatzstücks 100 im Bereich des Zwischenabschnitts 109 dar als jene der Fig. 1. Mit der Ausgestaltung der Fig. 3 kann dazu beigetragen werden, die Strömung durch die Hauptleitung 107 möglichst unberührt zu lassen. Auf diese Weise kann das Strömungsprofil der Strömung durch die Hauptleitung 107 möglichst unverändert belassen werden. So ist es einfacher, die zweite Flüssigkeit in einem Bereich höchster Strömungsgeschwindigkeit - bezogen auf die erste Flüssigkeit der Hauptleitung 107 - in diese einzubringen, nämlich in einem radial zentralen Bereich hiervon.

**Fig. 3a** zeigt das erfindungsgemäße Einsatzstück 100 der Fig. 3 in einem Querschnitt. Die Strömung durch die Hauptleitung 107 erfolgt in Fig. 3a hauptsächlich in die Zeichenebene hinein oder aus dieser heraus, also senkrecht zur Zeichenebene.

Die der Austrittsöffnung 115 gegenüber liegende Prallfläche oder Umlenkfläche 113' weist eine in der Ansicht der Fig. 3 nicht erkennbare Rille 113a auf. Die Rille 113a ist Teil des Lumenabschnitts 113 oder kann Teil hiervon sein. Sie erstreckt sich vorzugweise in der Richtung der Strömung durch die Hauptleitung 107 hindurch und steht somit zumindest abschnittsweise senkrecht zur Zeichenebene.

Die Rille 113a kann wie die Umlenkfläche 113' dazu dienen, dass aus der Austrittsfläche 115 austretende zweite Flüssigkeit bei ihrem Austritt aus der Austrittsfläche 115 nicht bis in radial außen gelegene Strömungsbereiche ("Stagnationsbereiche") der Hauptleitung 107 ausgebracht wird, sondern sich näher an der Mittellinie M_H, also in einem Bereich besonders hoher Strömungsgeschwindigkeit, in den Strom der ersten Flüssigkeit hinein begibt und in diesem bedingt durch den Verlauf der Rille 113a über eine vorbestimmte Strecke geführt wird.

Der Querschnitt der Rille 113a kann wie in Fig. 3a exemplarisch gezeigt, dreieckig sein. Andere Querschnittsformen sind ebenfalls von der vorliegenden Erfindung umfasst.

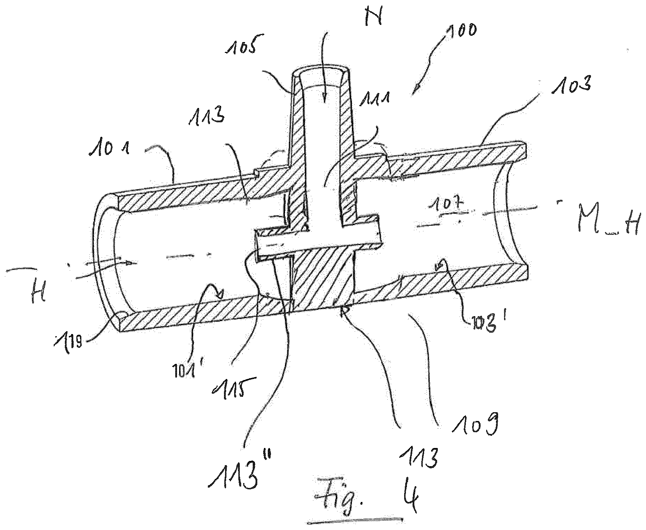

**Fig. 4** zeigt das erfindungsgemäße Einsatzstück 100 für einen in Fig. 4 nicht gezeigten Blutschlauchsatz 200 in einer dritten exemplarischen Ausführungsform in einem Längsschnitt.

Wie Fig. 4 zu entnehmen ist, umfasst der Lumenabschnitt 113 neben jenen Anteilen, die aus Fig. 3 bekannt sind, ferner einen in Längsrichtung der Hauptleitung 107 ausgerichteten Rohrabschnitt 113". Der Rohrabschnitt 113" kann eine runde Querschnittsform haben. Andere, beliebige Querschnittsformen kommen ebenfalls in Betracht und sind von der vorliegenden Erfindung ebenfalls umfasst.

In Fig. 4 deckt sich die Längsachse des Rohrabschnitts 113" mit der Längsrichtung bzw. hier der Mittellinie M_H der Hauptleitung 107. In anderen Ausführungsformen liegt die Längsachse des Rohrabschnitts 113" oder dessen Längsrichtung parallel zur Längsrichtung bzw. hier der Mittellinie M_H der Hauptleitung 107.

Andere als in Fig. 4 gezeigte Umlenkelemente zum Umlenken der aus der Nebenleitung 111 austretenden Flüssigkeit derart, dass sie möglichst mit paralleler Strömungsrichtung bereits aus der Austrittsöffnung 115 austritt und in die Strömung der durch den Hauptleitung 107 strömenden Flüssigkeit eintritt, sind ebenfalls von der vorliegenden Erfindung umfasst.

Das Umlenkelement 113" kann je nach Ausgestaltung vorteilhaft der Stabilisierung der Strömung dienen. Es kann vorteilhaft dazu beitragen, sicherzustellen, dass die zweite Flüssigkeit tatsächlich in einer Mitte des Strömungsprofils über den Querschnitt eingebracht wird, bzw. dort, wo die Strömungsgeschwindigkeit innerhalb der Hauptleitung 107 zumindest ausreichend hoch oder gar am höchsten ist.

Wie die Prall- oder Umlenkfläche 113 der Fig. 3 kann der Rohrabschnitt 113" dazu beitragen, den Austritt der zweiten Flüssigkeit aus der Austrittsöffnung 115 in einem Bereich erfolgen zu lassen, in welchem die Strömung in der Hauptleitung 107 nicht stagniert.

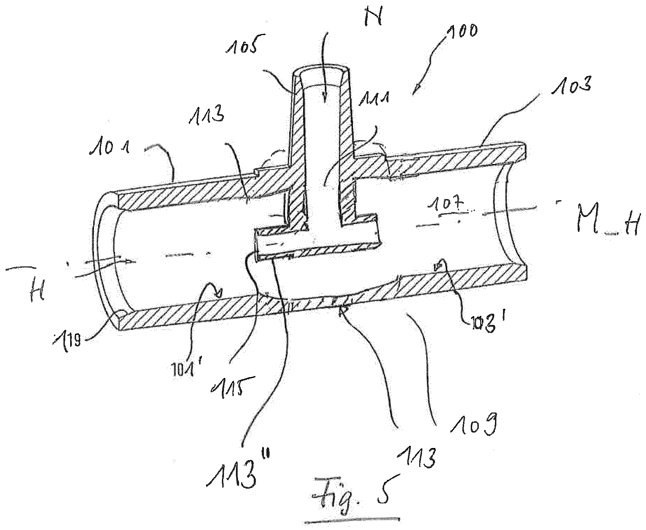

**Fig. 5** zeigt das erfindungsgemäße Einsatzstück 100 für einen in Fig. 3 nicht gezeigten Blutschlauchsatz 200 in einer vierten exemplarischen Ausführungsform in einem Längsschnitt.

Wie Fig. 5 zu entnehmen ist, umfasst der Lumenabschnitt 113 den Rohrabschnitt 113" der Fig. 4. Anders als in Fig. 4 reicht der Lumenabschnitt 113 jedoch nicht durch das gesamte Innere des Zwischenabschnitts 109. Der Lumenabschnitt 113 verringert daher das durchströmbare Lumen des Zwischenabschnitts 109 weniger als in Fig. 4.

Die vorstehenden Figuren zeigen Ausführungsformen, in welchen die zweite Flüssigkeit aus einer Austrittsöffnung 115 austritt. Von der Erfindung umfasst sind jedoch auch Lösungen mit mehr als nur einer Austrittsöffnung 115. So können mehrere, vor allem axial voneinander beabstandete Austrittsöffnungen vorgesehen sein.

**Fig. 6** zeigt eine optionale, grundsätzliche Anordnung eines erfindungsgemäßen Einsatzstücks 100 in einem schematisch sehr vereinfacht dargestellten, erfindungsgemäßen extrakorporalen Blutschlauchsatz 200 in einer exemplarischen Ausführungsform.

Der Blutschlauchsatz 200 weist einen Hämofilter 201 auf oder ist hiermit verbunden. Mit dem Hämofilter 201, oder Dialysator oder Blutfilter, sind eine Blutentnahmeleitung 203 (oder arterielle Leitung) und eine Blutrückgabeleitung 205 (oder venöse Leitung) verbunden.

Die Blutentnahmeleitung 203 ist mit einer Blutpumpe 301 in Wirkverbindung verbunden oder weist diese auf.

Stromauf der Blutpumpe 301 mündet eine weitere Zugabeleitung, hier eine Leitung 207 für Citratlösung, in die Blutentnahmeleitung 203.

Die Leitung 207 ist mit einer Citratpumpe 307 in Wirkverbindung verbunden oder weist diese auf.

Stromab des Hämofilters 201 mündet die erfindungsgemäße Leitung 209 für Calciumlösung in die Blutrückgabeleitung 205.

Das erfindungsgemäße Einsatzstück 100 ist mit einer Calciumpumpe 309 in Wirkverbindung verbunden oder weist diese auf. Sie wird aus einer in Fig. 1 nicht gezeigten Quelle für eine Infusionslösung, hier exemplarisch eine Calciumquelle, gespeist. Die Quelle kann ein Beutel oder eine Flasche sein. Optional kann die Infusionslösung on-line erzeugt sein; in diesem Fall zählt die entsprechende Vorrichtung, in welcher die Infusionslösung erzeugt wird, als Quelle.

Die Wirkverbindung mit der Calciumpumpe 309 ist hier als exemplarisch zu verstehen. Von der vorliegenden Erfindung mit umfasst ist eine Anordnung des erfindungsgemäßen Einsatzstücks 100 hinter einer anderen Pumpe als einer Calciumpumpe, also z. B. stromab einer Citratpumpe wie der Citratpumpe 307 der Fig. 6.

Der Hämofilter 201 ist mit einer Leitung 311 für frische Dialysierflüssigkeit und einer Leitung 315 für verbrauchtes Dialysat oder Filtrat verbunden. Die Leitung 311 ist mit einer Dialysierflüssigkeitspumpe 313 verbunden oder weist diese auf. Die Leitung 315 ist mit einer Filtratpumpe 317 verbunden oder weist diese auf.

Die gezeigten Pfeilspitze geben jeweils die Strömungsrichtung in einer bestimmungsgemäßen Benutzung des Blutschlauchsatzes 200 an.

Der in Fig. 6 gezeigte Blutschlauchsatz 200 kann einem üblichen extrakorporalen Blutschlauchsatz entsprechen und insbesondere für die CWHD (*continuous veno-venous hemodialysis*) geeignet sein.

Die Pumpen 301, 307, 309, 313 und 317 können Teil einer nur schematisch angedeuteten Blutbehandlungsvorrichtung 300 sein. Dasselbe gilt für die Leitungen 311 und 315.

### Bezugszeichenliste

- 100: Einsatzstück
- 101: erste Anschlussstelle
- 101': Innenwandung
- 103: zweite Anschlussstelle
- 103': Innenwandung
- 105: dritte Anschlussstelle
- 107: Hauptleitung
- 109: Zwischenabschnitt
- 109': Innenwandung
- 111: Nebenleitung
- 113: Lumenabschnitt
- 113': Prall- oder Umlenkfläche
- 113": Rohrabschnitt
- 113a: Rille
- 115: Austrittsöffnung oder Mündung
- 119: Abschrägung oder Fase

- 200: Blutschlauchsatz, Blutschlauchsystem
- 201: Hämofilter oder Blutfilter oder Dialysator
- 203: Blutentnahmeleitung
- 205: Blutrückgabeleitung
- 205a: erster Schlauchabschnitt
- 205b: zweiter Schlauchabschnitt
- 207: Leitung für Citratlösung
- 209: Leitung für Calciumlösung; Zugabeleitung; dritter Schlauchabschnitt

- 300: Blutbehandlungsvorrichtung
- 301: Blutpumpe
- 307: Citratpumpe
- 309: Calciumpumpe
- 311: Leitung für Dialysierflüssigkeit
- 313: Pumpe für Dialysierflüssigkeit
- 315: Leitung für Dialysat, Filtrat
- 317: Pumpe für Dialysat, Filtrat

- A: Wert
- a: Absatz
- D: Außendurchmesser der Nebenleitung
- d: Innendurchmesser der Nebenleitung
- H: Einströmrichtung des ersten Fluids in die Hauptleitung
- N: Einströmrichtung des zweiten Fluids in die Nebenleitung
- M_H: Mittellinie der Hauptleitung
- M_N: Mittellinie der Nebenleitung
- M_A M_115: Mittelpunkt einer Öffnungsebene der Austrittsöffnung
- r: radiale Richtung oder Erstreckung der Hauptleitung

## Patentansprüche

1. Einsatzstück (100) für einen Blutschlauchsatz (200), umfassend jeweils wenigstens
- eine erste Anschlussstelle (101) zum Anschließen eines ersten Schlauchabschnitts (205a) des Blutschlauchsatzes (200) an das Einsatzstück (100);
- eine zweite Anschlussstelle (103) zum Anschließen eines zweiten Schlauchabschnitts (205b) des Blutschlauchsatzes (200) an das Einsatzstück (100);
- eine dritte Anschlussstelle (105) zum Anschließen eines dritten Schlauchabschnitts (209) des Blutschlauchsatzes (200) an das Einsatzstück (100);
- eine Hauptleitung (107) zum Leiten einer ersten Flüssigkeit, vorzugsweise Blut, durch das Einsatzstück (100) hindurch, wobei die Hauptleitung (107) in Fluidverbindung steht mit der ersten Anschlussstelle (101) und mit der zweiten Anschlussstelle (103);
- eine Nebenleitung (111) zum Leiten einer zweiten Flüssigkeit, vorzugsweise einer Infusion, in die Hauptleitung (107) hinein, wobei die Nebenleitung (111) in Fluidverbindung steht mit der dritten Anschlussstelle (105), und wobei die Nebenleitung (111) ferner in einem Zwischenabschnitt (109) des Einsatzstücks (100), welcher zwischen der ersten Anschlussstelle (101) und der zweiten Anschlussstelle (103) angeordnet ist, in Fluidverbindung steht mit der Hauptleitung (107);
wobei:
- die erste Anschlussstelle (101) ein durchströmbares Lumen mit einer ersten Querschnittsfläche aufweist;
- die zweite Anschlussstelle (103) ein durchströmbares Lumen mit einer zweiten Querschnittsfläche aufweist;
- der Zwischenabschnitt (109) ein durchströmbares Lumen mit einer dritten Querschnittsfläche aufweist;
**dadurch gekennzeichnet, dass**
- die Nebenleitung (111) einen Lumenabschnitt (113) aufweist, welcher in ein Inneres des Zwischenabschnitts (109) ragt und welcher wenigstens eine Mündung oder Austrittsöffnung (115) für das zweite Fluid aufweist; und
- im Inneren des Einsatzstücks keine Strömungshindernisse vorhanden sind mit Ausnahme der Nebenleitung und/oder einer optionalen Prall- oder Umlenkfläche (113') im Bereich des Zwischenabschnitts (109), welche angeordnet ist, um die aus der Austrittsöffnung oder Mündung der Nebenleitung (111) austretende zweite Flüssigkeit in ihrer radialen Bewegung oder in ihrer Bewegung in Austrittsrichtung zu begrenzen.

2. Einsatzstück (100) nach einem der vorangegangenen Ansprüche, wobei die dritte Querschnittsfläche so groß oder im Wesentlichen so groß wie die erste Querschnittsfläche und/oder die zweite Querschnittsfläche ist.

3. Einsatzstück (100) nach einem der vorangegangenen Ansprüche, wobei der Lumenabschnitt (113) ausgestaltet ist, um die zweite Flüssigkeit in, oder im Wesentlichen in, einer axialen Richtung der Hauptleitung (107) oder parallel zu einer Hauptströmungsrichtung der Hauptleitung (107) in letztere abzugeben.

4. Einsatzstück (100) nach einem der vorangegangenen Ansprüche, wobei die Austrittsöffnung (115) angeordnet ist, um die zweite Flüssigkeit in, oder im Wesentlichen in, einer axialen Richtung der Hauptleitung (107) oder parallel zu einer Hauptströmungsrichtung der Hauptleitung (107) in letztere abzugeben.

5. Einsatzstück (100) nach einem der vorangegangenen Ansprüche, wobei ein sich an die Austrittsöffnung (115) stromaufwärts anschließender Abschnitt des Lumenabschnitts (113) derart angeordnet ist, dass sich seine Längsrichtung parallel, oder im Wesentlichen, parallel, zur Längsachse des Zwischenabschnitts (109) erstreckt.

6. Einsatzstück (100) nach einem der vorangegangenen Ansprüche, wobei im Bereich des Zwischenabschnitts (109) ein Prall- oder Umlenkelement (113') im Inneren des Zwischenabschnitts (109) derart angeordnet ist, dass eine aus der Austrittsöffnung (115) oder Mündung der Nebenleitung (111) austretende zweite Flüssigkeit in ihrer radialen Bewegung begrenzt ist.

7. Einsatzstück (100) nach einem der vorangegangenen Ansprüche, wobei sich der Lumenabschnitt (113), einstückig oder mehrstückig, in radialer Richtung (r) durch das gesamte Innere des Zwischenabschnitts (109) oder der Hauptleitung (107) erstreckt.

8. Einsatzstück (100) nach einem der vorangegangenen Ansprüche, wobei der Lumenabschnitt (113) wenigstens eine Rille (113a) aufweist, welche sich zumindest abschnittsweise in einer Richtung parallel zur Hauptleitung (107) erstreckt.

9. Einsatzstück (100) nach einem der vorangegangenen Ansprüche, wobei sich im Bereich des Zwischenabschnitts (109) ein Vorsprung in das Innere des Zwischenabschnitts (109) erstreckt, welcher in einer Verlängerung der Nebenleitung (111) im Zwischenabschnitt (109) liegt.

10. Blutschlauchsatz (200) oder extrakorporaler Blutkreislauf (200), welcher wenigstens ein Einsatzstück (100) nach einem der vorangegangenen Ansprüche aufweist.

11. Blutschlauchsatz (200) nach Anspruch 10, wobei das Einsatzstück (100) in Fluidkommunikation mit einer Zugabeleitung (207, 209) und in Fluidkommunikation mit einer Blutentnahmeleitung (203) und/oder einer Blutrückgabeleitung (205) verbunden ist.

12. Blutschlauchsatz (200) nach einem der Ansprüche 10 und 11, wobei der Blutschlauchsatz (200) für die Durchführung einer regionalen Antikoagulation geeignet und/oder vorbereitet ist.

13. Blutschlauchsatz (200) nach einem der Ansprüche 10 bis 12, wobei der Blutschlauchsatz (200) zur Durchführung einer Hämodialyse, einer Hämofiltration, einer Hämodiafiltration, einer Plasmapheresebehandlung oder Vollblutadsorptionsbehandlung geeignet ist.

14. Blutschlauchsatz (200) nach einem der Ansprüche 10 bis 13, wobei die erste Anschlussstelle (101) und die zweite Anschlussstelle (103) mit einer blutführenden Leitung (205) des Blutschlauchsatzes (200) verbunden sind und die dritte Anschlussstelle (105) mit einer Leitung für eine Elektrolytlösung, insbesondere eine Calciumleitung, verbunden ist.

15. Blutbehandlungsvorrichtung (300), verbunden mit wenigstens einem Blutschlauchsatz (200) nach einem der Ansprüche 10 bis 14.

## Claims

1. An insert piece (100) for a blood tubing set (200), comprising at least, respectively:
- a first connection site (101) for connecting a first tubing portion (205a) of the blood tubing set (200) to the insert piece (100);
- a second connection site (103) for connecting a second tubing portion (205b) of the blood tubing set (200) to the insert piece (100);
- a third connection site (105) for connecting a third tubing portion (209) of the blood tubing set (200) to the insert piece (100);
- a main line (107) for conducting a first liquid, preferably blood, through the insert piece (100), the main line (107) being in fluid communication with the first connection site (101) and with the second connection site (103);
- a secondary line (111) for conducting a second liquid, preferably an infusion, into the main line (107),
the secondary line (111) being in fluid communication with the third connection site (105), and
the secondary line (111) being also in fluid communication with the main line (107) in an intermediate portion (109) of the insert piece (100) being arranged between
the first connection site (101) and the second connection site (103);
wherein
- the first connection site (101) comprises a flow-through lumen having a first cross section area;
- the second connection site (103) comprises a flow-through lumen having a second cross section area;
- the intermediate portion (109) comprises a flow-through lumen having a third cross section area;
**characterized in that**:
- the secondary line (111) comprises a lumen portion (113) which protrudes into an interior of the intermediate portion (109) and which comprises at least an outlet or outlet opening (115) for the second fluid; and
- no flow obstacles are present inside the insert piece, with the exception of the secondary line and/or of an optional baffle or deflector element (113') arranged in the area of the intermediate portion (109) to limit the second liquid flowing out of the outlet opening or of the outlet of the secondary line (111) in its radial movement or in its movement in the outlet direction.

2. The insert piece (100) according to claim 1, wherein the third cross section area is as large, or substantially as large, as the first cross section area and/or the second cross section area.

3. The insert piece (100) according to anyone of the preceding claims, wherein the lumen portion (113) is designed to deliver the second liquid into, or substantially into, the main line (107), in an axial direction of the main line (107) or parallel to a main flow direction of the main line (107).

4. The insert piece (100) according to anyone of the preceding claims, wherein the outlet opening (115) is arranged to deliver the second liquid into, or substantially into, the main line (107), in an axial direction of the main line (107) or parallel to a main flow direction of the main line (107).

5. The insert piece (100) according to anyone of the preceding claims, wherein a portion of the lumen portion (113) adjoining the outlet opening (115) upstream of the latter is arranged such that its longitudinal direction extends parallel, or substantially parallel, to the longitudinal axis of the intermediate portion (109).

6. The insert piece (100) according to anyone of the preceding claims, wherein in the area of the intermediate portion (109) a baffle or deflector element (113') is arranged in the interior of the intermediate portion (109) such that a second liquid flowing out of the outlet opening (115) or of the outlet of the secondary line (111) is limited in its radial movement.

7. The insert piece (100) according to anyone of the preceding claims, wherein the lumen portion (113) extends in one piece or in multiple pieces, through the entire interior of the intermediate portion (109) or of the main line (107) in radial direction (r).

8. The insert piece (100) according to anyone of the preceding claims, wherein the lumen portion (113) comprises at least one groove (113a) which extends, at least in sections, in a direction parallel to the main line (107).

9. The insert piece (100) according to anyone of the preceding claims, wherein a protrusion extends into the interior of the intermediate portion (109) in the area of the intermediate portion (109), which protrusion lies in an extension of the secondary line (111) in the intermediate portion (109).

10. A blood tubing set (200) or an extracorporeal blood circuit (200), which comprises at least one insert piece (100) according to anyone of the preceding claims.

11. The blood tubing set (200) according to claim 10, wherein the insert piece (100) is connected in fluid communication with an addition line (207, 209) and in fluid communication with a blood withdrawal line (203) and/or a blood return line (205).

12. The blood tubing set (200) according to anyone of claims 10 and 11, wherein the blood tubing set (200) is suitable and/or prepared for executing a regional anticoagulation.

13. The blood tubing set (200) according to anyone of claims 10 to 12, wherein the blood tubing set (200) is suitable for executing a hemodialysis, a hemofiltration, a hemodia-filtration, a plasmapheresis treatment or a whole blood adsorption treatment.

14. The blood tubing set (200) according to anyone of claims 10 to 13, wherein the first connection site (101) and the second connection site (103) are connected to a blood conducting line of the blood tubing set (200) and the third connection site (105) is connected to a line for an electrolyte solution, in particular a calcium line.

15. A blood treatment apparatus (300), connected with at least one blood tubing set (200) according to anyone of claims 10 to 14.

## Revendications

1. Une pièce d'insertion (100) pour un set de tuyaux sanguins (200), comprenant au moins, respectivement:
- un premier point de raccordement (101) pour raccorder une première section de tuyau (205a) du set de tuyaux sanguins (200) à la pièce d'insertion (100);
- un second point de raccordement (103) pour raccorder une seconde section de tuyau (205b) du set de tuyaux sanguins (200) à la pièce d'insertion (100);
- un troisième point de raccordement (105) pour raccorder une troisième section de tuyau (209) du set de tuyaux sanguins (200) à la pièce d'insertion (100);
- un conduit principal (107) pour acheminer un premier liquide, de préférence du sang, au travers de la pièce d'insertion (100), le conduit principal (107) étant en communication fluidique avec le premier point de raccordement (101) ainsi qu'avec le second point de raccordement (103);
- un conduit secondaire (111) pour acheminer un second liquide, de préférence une infusion, dans le conduit principal (107), le conduit secondaire (111) étant en communication fluidique avec le troisième point de raccordement (105), et le conduit secondaire (111) étant en outre en communication fluidique avec le conduit principal (107) dans une section intermédiaire (109) de la pièce d'insertion (100) agencée entre le premier point de raccordement (101) et le second point de raccordement (103);
où
- le premier point de raccordement (101) comprend une lumière pouvant être traversée ayant une première de section transversale;
- le second point de raccordement (103) comprend une lumière pouvant être traversée ayant une seconde section transversale;
- la section intermédiaire (109) comprend une lumière pouvant être traversée ayant une troisième section transversale;
**caractérisée en ce que**:
- le conduit secondaire (111) comprend une section de lumière (113) qui fait saillie à l'intérieur de la section intermédiaire (109) et qui comprend au moins une embouchure ou un orifice de sortie (115) pour le second fluide; et
- **en ce qu'**aucun obstacle à l'écoulement n'est présent à l'intérieur de la pièce d'insertion, à l'exception du conduit secondaire et/ou d'une chicane ou d'un élément déflecteur optionnel(le) (113') agencé (e) dans la zone de la section intermédiaire (109) pour limiter le second liquide s'écoulant de l'orifice de sortie ou de l'embouchure du conduit secondaire (111) dans son mouvement radial ou dans son mouvement allant dans le sens de sortie.

2. La pièce d'insertion (100) selon la première revendication, où la troisième section transversale est aussi grande, ou essentiellement aussi grande, que la première section transversale et/ou la seconde section transversale.

3. La pièce d'insertion (100) selon l'une quelconque des revendications précédentes, où la section de lumière (113) est conçue pour délivrer le second liquide dans, ou essentiellement dans, le conduit principal (107), dans une direction axiale du conduit principal (107) ou parallèlement à une direction d'écoulement principale du conduit principal (107).

4. La pièce d'insertion (100) selon l'une quelconque des revendications précédentes, où l'orifice de sortie (115) est agencé pour délivrer le second liquide dans, ou essentiellement dans, le conduit principal (107), dans une direction axiale du conduit principal (107) ou parallèlement à une direction d'écoulement principale du conduit principal (107).

5. La pièce d'insertion (100) selon l'une quelconque des revendications précédentes, où une portion de la section de lumière (113) jouxtant en amont l'orifice de sortie (115) est agencée de telle sorte que sa direction longitudinale s'étend parallèlement, ou essentiellement parallèlement, à l'axe longitudinal de la section intermédiaire (109).

6. La pièce d'insertion (100) selon l'une quelconque des revendications précédentes, où une chicane ou un élément déflecteur (113') est agencé(e) à l'intérieur de la section intermédiaire (109) dans la zone de la section intermédiaire (109), de telle sorte qu'un second liquide s'écoulant de l'orifice de sortie (115) ou de l'embouchure du conduit secondaire (111) est limité dans son mouvement radial.

7. La pièce d'insertion (100) selon l'une quelconque des revendications précédentes, où la section de lumière (113) s'étend, en une seule pièce ou en plusieurs pièces, au travers de tout l'intérieur de la section intermédiaire (109) ou du conduit principal (107) dans une direction radiale (r).

8. La pièce d'insertion (100) selon l'une quelconque des revendications précédentes, où la section de lumière (113) comprend au moins une rainure (113a) qui s'étend, au moins par portions, dans une direction parallèle au conduit principal (107).

9. La pièce d'insertion (100) selon l'une quelconque des revendications précédentes, où une saillie s'étend à l'intérieur de la section intermédiaire (109) dans la zone de la section intermédiaire (109), laquelle saillie se situe dans un prolongement du conduit secondaire (111) dans la section intermédiaire (109).

10. Un set de tuyaux sanguins (200) ou un circuit sanguin extracorporel (200), qui comprend au moins une pièce d'insertion (100) selon l'une quelconque des revendications précédentes.

11. Le set de tuyaux sanguins (200) selon la revendication 10, où la pièce d'insertion (100) est reliée en communication fluidique avec un conduit d'addition (207, 209) et en communication fluidique avec un conduit de prélèvement sanguin (203) et/ou un conduit de retour sanguin (205).

12. Le set de tuyaux sanguins (200) selon l'une quelconque des revendications 10 et 11, où le set de tuyaux sanguins (200) est adapté et/ou préparé pour réaliser une anticoagulation régionale.

13. Le set de tuyaux sanguins (200) selon l'une quelconque des revendications 10 à 12, où le set de tuyaux sanguins (200) est adapté pour réaliser une hémodialyse, une hémofiltration, une hémodiaflitration, un traitement par plasmaphérèse ou un traitement par adsorption de sang total.

14. Le set de tuyaux sanguins (200) selon l'une quelconque des revendications 10 à 13, ou le premier point de raccordement (101) et le second point de raccordement (103) sont reliés à un conduit d'acheminement du sang du set de tuyaux sanguins (200) et où le troisième point de raccordement (105) est relié à un conduit pour une solution électrolytique, notamment un conduit de calcium.

15. Un appareil de traitement du sang (300) relié à au moins un set de tuyaux sanguins (200) selon l'une quelconque des revendications 10 à 14.
